# EUROPÄISCHE PATENTSCHRIFT

(11) **EP 2 558 861 B1**
(45) Veröffentlichungstag und Bekanntmachung des Hinweises auf die Patenterteilung: **10.08.2016**
(21) Anmeldenummer: 11714312.3
(22) Anmeldetag: 14.04.2011
(51) Int. Cl.: G01N 33/564, G01N 33/50

(54) **VERFAHREN UND MITTEL ZUR IDENTIFIZIERUNG VON SUBSTANZEN, WELCHE DIE IgE-PRODUKTION HEMMEN**
METHOD AND MEANS OF IDENTIFICATION OF SUBSTANCES, WHICH INHIBIT IGE PRODUCTION
METHODE ET DISPOSITIFS DE L'IDENTIFICATION DE SUBSTANCES, QUI INHIBENT LA PRODUCTION DE IGE

(30) Priorität: 15.04.2010 DE 102010027827
(43) Veröffentlichungstag der Anmeldung: 20.02.2013
(73) Patentinhaber: THORNE LIMITED, Vancouver, V6Z1S4 (CA)
(72) Erfinder: Jessberger, Rolf, 01326 Dresden (DE); Audzevich, Dr., Tatsiana, London WC1E 6JJ (GB)
(74) Vertreter: Kailuweit & Uhlemann Patentanwälte Partnerschaft mbB
(86) Internationale Anmeldenummer: PCT/EP2011/055942
(87) Internationale Veröffentlichungsnummer: WO 2011/141267

(56) Entgegenhaltungen:
- BORGGREFE T ET AL: "Impaired IgE response in SWAP-70-deficient mice.", EUROPEAN JOURNAL OF IMMUNOLOGY AUG 2001 LNKD- PUBMED:11500831, Bd. 31, Nr. 8, August 2001 (2001-08), Seiten 2467-2475, XP002641607, ISSN: 0014-2980
- SIVALENKA RAJA R ET AL: "SWAP-70 regulates mast cell FcepsilonRI-mediated signaling and anaphylaxis.", EUROPEAN JOURNAL OF IMMUNOLOGY MAR 2008 LNKD- PUBMED:18236401, Bd. 38, Nr. 3, März 2008 (2008-03), Seiten 841-854, XP002641608, ISSN: 0014-2980
- HELLMAN ET AL: "Regulation of IgE homeostasis, and the identification of potential targets for therapeutic intervention", BIOMEDICINE AND PHARMACOTHERAPY, ELSEVIER, PARIS, FR, Bd. 61, Nr. 1, 2. Februar 2007 (2007-02-02), Seiten 34-49, XP005870867, ISSN: 0753-3322, DOI: DOI:10.1016/J.BIOPHA.2006.10.001
- OETTGEN H C: "Regulation of the IgE isotype switch: new insights on cytokine signals and the functions of @? germline transcripts", CURRENT OPINION IN IMMUNOLOGY, ELSEVIER, OXFORD, GB, Bd. 12, Nr. 6, 1. Dezember 2000 (2000-12-01), Seiten 618-623, XP004257733, ISSN: 0952-7915, DOI: DOI:10.1016/S0952-7915(00)00153-9

### SEQUENCE LISTING

<110> Technische Universität Dresden
<120> Verfahren und Mittel zur Identifizierung von Substanzen, welche die IgE-Produktion hemmen
<130> 00017P0150DEWO
<150> DE 102010027827
   <151> 2010-04-15
<160> 18
<170> PatentIn version 3.3
<210> 1
   <211> 88
   <212> DNA
   <213> Mus musculus
<400> 1
   cccactgtgc cttagtcaac ttcccaagaa cagaatcaaa agggaacttc caaggctgct    60
   aaggccgggg gttcccaccc cactttta                                      88
<210> 2
   <211> 17
   <212> DNA
   <213> Mus musculus
<400> 2
   tgtctcctag aaagagg 17
<210> 3
   <211> 20
   <212> DNA
   <213> Artificial
<220>
   <223> Primer
<400> 3
   tcgagaagcc tgaggaatgt 20
<210> 4
   <211> 20
   <212> DNA
   <213> Artificial
<220>
   <223> Primer
<400> 4
   atagacagat gggggtgtcg 20
<210> 5
   <211> 20
   <212> DNA
   <213> Artificial
<220>
   <223> Primer
<400> 5 20
   ctggccagcc actcacttat 20
<210> 6
   <211> 20
   <212> DNA
   <213> Artificial
<220>
   <223> Primer
<400> 6 20
   cagtgccttt acagggcttc 20
<210> 7
   <211> 20
   <212> DNA
   <213> Artificial
<220>
   <223> Primer
<400> 7 20
   cacaggacta gaacacctgc 20
<210> 8
   <211> 16
   <212> DNA
   <213> Artificial
<220>
   <223> Primer
<400> 8 16
   gctggtgaag acctct 16
<210> 9
   <211> 21
   <212> DNA
   <213> Artificial
<220>
   <223> Primer
<400> 9 21
   ctagaaagag gcctacacct g 21
<210> 10
   <211> 18
   <212> DNA
   <213> Artificial
<220>
   <223> Primer
<400> 10
   ccagactgtt cttattcg 18
<210> 11
   <211> 20
   <212> DNA
   <213> Artificial
<220>
   <223> Primer
<400> 11
   agggggtgag ggggagtcca 20
<210> 12
   <211> 19
   <212> DNA
   <213> Artificial
<220>
   <223> Primer
<400> 12
   cccccaaagg cccaggtgc 19
<210> 13
   <211> 22
   <212> DNA
   <213> Artificial
<220>
   <223> Primer
<400> 13
   tggcatcgct gactctccaa ca 22
<210> 14
   <211> 20
   <212> DNA
   <213> Artificial
<220>
   <223> Primer
<400> 14
   ctgggtggcc acagcacaca 20
<210> 15
   <211> 21
   <212> DNA
   <213> Artificial
<220>
   <223> Primer
<400> 15
   tttgaactgg agctcagctg g 21
<210> 16
   <211> 21
   <212> DNA
   <213> Artificial
<220>
   <223> Primer
<400> 16
   tccatggtgc tagccatatg c 21
<210> 17
   <211> 22
   <212> DNA
   <213> Artificial
<220>
   <223> Primer
<400> 17
   ctgtcaccca ctttcaatcc tg 22
<210> 18
   <211> 23
   <212> DNA
   <213> Artificial
<220>
   <223> Primer
<400> 18
   gcgtgaagaa gattgctgct att 23

## Beschreibung

Die Erfindung betrifft das Gebiet der biomedizinischen und pharmakologischen Forschung, insbesondere im Bereich Immunologie, Allergien und Autoimmunkrankheiten.

### STAND DER TECHNIK

Allergien, darunter auch Asthma, und Autoimmunkrankheiten sind weltweit sehr verbreitete, zunehmende Erkrankungen, wobei Allergien in schweren Fällen bis zum Tod führen können. Die Inzidenz an Allergien hat sich in den letzten 20 Jahren verdreifacht. Zwischen 5 und 10 % der Weltbevölkerung leiden an Allergien, inklusive Asthma, an dem etwa ein Fünftel dieser Personen leiden. Asthma ist die häufigste chronische Erkrankung bei unter 18-Jährigen. Allergien werden durch übermässige IgE-Produktion der B-Lymphozyten ausgelöst. Eingriffe in die Regulation dieser IgE-Produktion sind somit prinzipiell wegweisend für eine ursächliche Allergietherapie, die bislang nicht existiert. Jedoch ist bisher zuwenig bekannt, wie die IgE-Produktion im Einzelnen reguliert wird.
Die IgE-Produktion hängt, wie seit längerem bekannt, von der die Transkription aktivierenden Funktion des Proteins STAT-6 ab. Es ist auch bekannt, dass der Effekt von STAT-6 auf die IgE-Produktion durch BCL-6 inhibiert wird. BCL-6 und STAT-6 binden einen überlappenden Bereich der regulatorischen DNA-Sequenz des IgE-Gens. Es wird angenommen, dass BCL-6 mit Co-Repressoren interagiert und dadurch aktiv die IgE-Produktion hemmt. Diese Repression ist spezifisch für das IgE-Gen, da BCL-6 selektiv an die STAT-6-Bindungstelle im IgE-Gen bindet und an die meisten anderen STAT-6-Bindungsstellen nicht bindet. Die exakten Mechanismen der Spezifität und der Regulation der kritischen Balance zwischen BCL-6 und STAT-6 sind jedoch nicht bekannt.

Das SWAP-70 Protein wird hauptsächlich in aktivierten B-Zellen, in Mastzellen, und in dendritischen Zellen exprimiert, obwohl die Präsenz des Proteins im Zytoplasma anderer Zelltypen nicht ausgeschlossen werden kann (Borggrefe et al.1998; Borggrefe et al. 1999; Masat et al. 2000; Gross et al, 2002; Pearce et al., 2006; Ocana-Morgner et al., 2009). Es ist bekannt, dass SWAP-70 defiziente Mäuse spezielle Veränderungen des Phenotyps aufweisen, u. a. sind die CD40-vermittelte Aktivierung von B-Zellen einschließlich dem Wechsel zur Produktion von IgE, und die Degranulierung von Mastzellen stark reduziert (Borggrefe et al., 2001; Gross et al., 2002). Hinsichtlich der IgE-Produktion in Abhängigkeit von SWAP-70 wurde gezeigt, dass in SWAP-70 defizienten Mäusen vor und nach Immunisierung stark verminderte IgE-Konzentrationen vorliegen, und dass die Produktion sekretierten IgEs in B-Zell-Kulturen um ca. das 8-fache reduziert ist. Die Produktion anderer Immunoglobulin-Klassen ist jedoch nicht wesentlich beeinträchtigt.

In US6,528,284 B1 (US2003/143611A1) wird auf die Reinigung und Charakterisierung von Proteinen, die generell am Immunoglobulin-Klassenwechsel beteiligt sind eingegangen. Identifiziert wurde SWAP-70, welches hier als SRTA-70 (S-Region Transfer Activity-70) bezeichnet und beansprucht wird. Beschrieben wird dessen allgemeine Rolle im Immunoglobulin-Klassenwechsel, sowie die Nutzung dieses Proteins in der Modulation des Klassenwechsels.

US 2003/0166018 A1 beschreibt die Rolle von SWAP-70 in der Mastzelldegranulation und beansprucht eine Methode zur Identifzierung eines Agens, welches den Gehalt von SWAP-70 Protein in einer Mastzelle reduziert.

Die Allergiebehandlung erfolgt heutzutage überwiegend mit Hilfe von Symptombehandlung, d.h. Linderung der allergischen Reaktion beispielsweise durch anti-Histaminika oder Glucocorticoide, nicht aber durch ursächliche Therapie. Nachteile bisheriger Therapien sind u. a. kurze Wirkung, keine Ursachenbehebung, unspezifische Wirkung und Nebenwirkungen.

Der einzige bisher bekannte IgE-Hemmer ist Omalizumab, ein rekombinanter humanisierter monoklonaler Antikörper gegen Immunglobulin E (Anti-IgE) zur Behandlung von schwerem allergischen Asthma bronchiale. Aber auch Omalizumab unterbindet nicht ursächlich die IgE-Produktion, sondern fängt vielmehr bereits produziertes IgE ab, wodurch Immunglobulinkomplexe entstehen. Nachteilig können Antikörpertherapien selbst allergische Reaktionen bzw. das Antikörperprotein attackierende Immunreaktionen auslösen. Zudem sind Antikörper pharmakologisch aufwändig herzustellen.

### AUFGABE DER ERFINDUNG

Aufgabe der Erfindung ist es daher ein Verfahren anzugeben, welches es erlaubt gezielt nach Wirkstoffen zu screenen, die als neue Anti-Allergika verwendet werden können und welche insbesondere die Produktion von IgE hemmen. Des Weiteren soll die Erfindung Mittel zur Durchführung des Screeningverfahrens angeben.

### BESCHREIBUNG DER ERFINDUNG

Die Erfindung beruht auf der Erkenntnis, dass SWAP-70 die Funktion von STAT-6 spezifisch in der IgE-Produktion positiv reguliert. In Abwesenheit von SWAP-70 findet sich eine stark reduzierte IgE-Produktion, wie die Erfinder *in vitro* und im Mausmodell zeigen konnten. Daher könnte die therapeutische Hemmung von SWAP-70 einen wichtigen Weg der Allergietherapie darstellen.

Eine weitere wichtige Erkenntnis ist, dass SWAP-70 die von BCL-6 ausgeübte Hemmung der IgE-Produktion negativ reguliert. In Abwesenheit von SWAP-70 bindet deutlich mehr BCL-6 an die relevante IgE DNA-Region, d.h. die IgE-Produktion wird gehemmt.

Die Wirkungen von SWAP-70 auf STAT-6 und auf BCL-6 sowie auf die für die IgE Produktion kritische Balance zwischen STAT-6 und BCL-6 waren bisher nicht bekannt.

Zusammengefasst lässt sich schlussfolgern, dass SWAP-70 die IgE-Produktion positiv reguliert, in dem es die Wechselwirkung der beiden Gegenspieler STAT-6 und BCL-6 mit regulatorischen Elementen des IgE Gens zugunsten des Aktivators STAT-6 verschiebt.

Die Erfinder haben gezeigt, dass SWAP-70 ein übergeordneter Regulator für STAT-6 und BCl-6 ist, der spezifisch für die IgE-Produktion in B-Zellen ist, in dem er den Effekt von STAT-6 verstärkt und den Effekt von BCL-6 antagonisiert. Die Erfinder haben keinen Einfluss von SWAP-70 auf die Expression von anderen Genen beobachtet, die ebenfalls durch STAT-6 und/oder BCL-6 reguliert werden.

Basierend auf diesen Erkenntnissen stellt die Erfindung ein Screeningverfahren zur Verfügung welches erlaubt neue Wirkstoffe zu identifizieren, welche spezifisch die IgE-Produktion hemmen.

Die Hemmung der Wirkung von SWAP-70 auf STAT-6 ist vorteilhaft hoch spezifisch für die IgE-Produktion in B-Zellen. Dies ermöglicht es, Wirkstoffe zu entwickeln, die IgE abhängige Allergien und Autoimmunkrankheiten nicht wie bisher symptomatisch, sondern ursächlich behandeln. Dies wird insbesondere durch Wirkstoffe erreicht, welche die stimulierende Funktion des SWAP-70 auf STAT-6 hemmen.

Das erfindungsgemäße Verfahren gemäß Anspruch 1 umfasst folgende Schritte:
(i) Inkontaktbringen:
   (a) mindestens einer Testsubstanz mit
   (b) SWAP-70 und
   (c) BCL-6 und/oder STAT-6
(ii) Bestimmung der Interaktion der Bindungspartner *in vitro, in situ* oder *in vivo,* wobei bei Messung der Interaktion in Vertebraten der Mensch ausgenommen ist,
(iii) Auswahl einer Testsubstanz, welche bevorzugt die SWAP-70 medierte Aktivierung von STAT-6 spezifisch inhibiert und/oder die BCL-6 medierte Inhibierung von STAT-6 verstärkt.

Bevorzugt wird in Schritt (iii) eine Testsubstanz ausgewählt, welche die Interaktion von SWAP-70 mit BCL-6 verstärkt und/oder so beeinflusst, so dass die inhibitorische Wirkung von BCL-6 verstärkt wird.

Alternativ wird eine Testsubstanz ausgewählt wird, welche die Interaktion von SWAP-70 mit STAT-6 hemmt.

Unter Interaktion wird dabei jeweils bevorzugt eine direkte Protein-Protein Wechselwirkung oder auch eine indirekte Interaktion verstanden, welche durch andere Proteine vermittelt wird.

Bevorzugt wird eine Testsubstanz ausgewählt wird, welche die SWAP-70 medierte Aktivierung von STAT-6 spezifisch inhibiert und/oder die BCL-6 medierte Inhibierung der SWAP-70 medierten Aktivierung von STAT-6 verstärkt bzw. den inhibierenden Effekt von SWAP-70 auf BCL-6 blockiert.

Die Testsubstanzen sind bevorzugt sogenannte "small molecules", d. h. Verbindungen mit einem Molekulargewicht bis 800 Daltons (atomare Masseneinheit u). Die Testsubstanzen sind bevorzugt organische Moleküle oder auch Peptide.

Die Interaktion der Proteine SWAP-70, STAT-6 und/oder BCL-6 und ggf. der regulatorischen Elemente des IgE Gens wird bevorzugt *in vitro* oder *in situ* gemessen.

*In vitro* (d. h. in zellfreien Verfahren) wird die Wirkung der Testsubstanz(en) auf die Interaktion zwischen SWAP-70 und BCL-6 und/oder SWAP-70 und STAT-6 (nachfolgend zusammen auch als Bindungspartner bezeichnet) bevorzugt unter Benutzung der entsprechenden gereinigten Proteine als Bindungspartner getestet. Dies erfolgt beispielsweise in Bindungsassays, in denen die Assoziation eines markierten mit einem unmarkierten oder zweier verschieden markierter Bindungspartner gemessen werden. Tests dieser Art können u. a. im Multiwell-Verfahren (ähnlich einem ELISA) oder in Lösung (z. B. mittels Alpha-Screen) im high throughput betrieben werden. Marker sind z. B. fluoreszierende Farbstoffe sein, die kovalent an einen oder an beide Bindungspartner gebunden werden. Alternativ erfolgt die Markierung radioaktiv, mittels Affinitätstags, Haptameren oder durch Markierung mit fluoreszierenden Proteinen (bevorzugt als Fusionsprotein - z. B. mit GFP). Das Verbleiben der Fluoreszenzsignale am unmarkierten Partner oder die Fluoreszenzinterferenz zwischen assoziierten Partnern wird ausgelesen.

*In situ* (d. h. in Zellen, bevorzugt in Zellkultur) wird die Wirkung der Testsubstanz(en) auf die Interaktion zwischen SWAP-70 und BCL-6 und/oder SWAP-70 und STAT-6 bevorzugt in Zellen gemessen welche SWAP-70 und BCL-6 und/oder SWAP-70 und STAT-6 exprimieren. Die Detektion der Interaktion zwischen den Bindungspartnern SWAP-70 und BCL-6 und/oder SWAP-70 und STAT-6 erfolgt hier bevorzugt in Zell-Lysaten. Auch hier erfolgt die Markierung der einzelnen Bindungspartner (SWAP-70, BCL-6 und/oder STAT-6) z. B. mittels AffinitätsTags (z. B. His-tag) und entsprechenden Affinitätsbindungsmaterialien (wie z. B. Ni²⁺) an diese Materialien gebunden.
Die Wirkung von zellgängigen Testsubstanzen beeinflusst die Bindungspartner ähnlich wie oben bei den *in vitro-* Methoden beschrieben. Bevorzugt werden die *in situ-* Verfahren mit Hilfe von gängigen, leicht kultivierbaren und transfizierbaren Zell-Linien, in denen die Bindungspartner exprimiert werden, durchgeführt. Geeignete Zelllinien sind z. B. NIH3T3, 293T und COS-7. Interaktionen zwischen den Bindungspartnern können vorteilhaft auch in Nicht-Säugerzellsystem analysiert werden, z. B. in Hefezellen (Yeast2 Hybrid-System) oder Insektenzellen (Co-Präzipitation nach Expression), oder nach Co-Translation in zellfreien Systemen (in vitro Transkriptions/Translationssysteme, gefolgt von Co-Präzipitationen, sog. pull-downs). Als negative Kontrollen werden bevorzugt entsprechende Zellen oder Zelllinien verwendet in denen die Gene, die für einzelne dieser Proteine codieren, nicht vorhanden sind oder deletiert sind (z. B. Zellen aus SWAP-70^{-/-} Mäusen, wie z. B. embryonale Mausfibroblasten). Multiwell-Formate erlauben high throughput screening Verfahren. Alternativ kann die Detektion der Interaktion der Bindungspartner *in vitro* oder auch in der Zelle durch Förster-Resonanzenergietransfer (FRET)-Analysen untersucht werden. Dazu werden zwei der Bindungspartner (vorzugsweise SWAP-70 und BCL-6 oder alternativ SWAP-70 und STAT-6) bevorzugt mit zwei zueinander unterschiedlichen Molekülen, die zur Erzeugung eines FRET geeignet sind, markiert. Diese beiden zueinander unterschiedlichen Moleküle werden nachfolgend auch FRET-Donor- und FRET-Akzeptor (oder auch einfach Donor und Akzeptor) genannt.

Der Förster-Resonanzenergietransfer (kurz FRET) ist ein physikalischer Prozess, bei dem Energie eines angeregten Donor strahlungsfrei auf ein Akzeptor im Abstand von etwa 1,5 bis 10 nm übertragen werden kann. Die Moleküle sind dazu so ausgewählt, dass das Emissionsspektrum des Donormoleküls mit dem Anregungsspektrum des Akzeptor überlappt. Sind sowohl Donor als auch Akzeptor Fluoreszenzfarbstoffe, spricht man von einem Fluorescence resonance energy transfer. Ist der Donor oder Akzeptor hingegen eine Chemilumineszenz- oder eine Biolumineszenzquelle wird der Begriff Chemilumineszenz-Resonanzenergietransfer (CRET) bzw. Biolumineszenz-Resonanzenergietransfer (BRET) verwendet.

Für *in vitro* Assays wird der Donor und/oder der Akzeptor bevorzugt an den Bindungspartner kovalent oder durch Koordination gebunden. Die Markierung des Proteins erfolgt bevorzugt kovalent an freie Aminogruppen oder Carboxylgruppen der Aminosäurenseitenketten (Lysin- oder Aspartat- oder Glutamat-Reste) des Proteins. Bevorzugt werden in der Erfindung als Donoren und Akzeptoren organische oder anorganische Fluoreszenzfarbstoffe verwendet. Bevorzugt sind pro Proteinmolekül 1 bis 40, besonders bevorzugt 1 bis 5 Farbstoffmoleküle gebunden. Als anorganische Fluoreszenzfarbstoffe werden z. B. Europium (bevorzugt Eu³⁺), Cer oder Terbium oder sogenannte Quantum dots (Quantenpunkte), wie z.B. dotierte LaF₃ und LaPO₄-Nanopartikel, verwendet.

Die Anbindung eines Biomoleküls, wie fluoreszierenden Proteinen (z. B. GFP, YFP und CFP) und/oder Luciferase erfolgt entweder durch Crosslinking oder durch Herstellung eines Fusionsproteins. Fusionsproteine mit derartigen Biomolekülen erlauben vorteilhaft auch die Detektion der Interaktion der Bindungspartner *in situ* (in der Zelle). Lumineszenzquellen (wie Luciferase) und fluoreszierende Proteine können vorteilhaft als Akzeptoren und Donoren eingesetzt werden.

Alternativ zu Zellen können auch multizelluläre Organismen. Hier wird die Interaktion der Bindungspartner bevorzugt *in vivo* bestimmt. Andere multizelluläre Organismen, sind ausgewählt aus Vertebraten (Mensch ausgenommen) und Invertebraten, wie *Drosophila melanogaster, Caenorhabditis elegans, Xenopus laevis,* Medaka, Zebrafish oder *Mus musculus,* oder deren Embryonen.

Bevorzugte Donor-Akzeptorpaare und ihre jeweiligen Anregungsmaxima (Maxₑₓ) sowie Emissionsmaxima (Maxₑₘ) sind ausgewählt aus:

| **Donor** | | | **Akzeptor** | | |
|---|---|---|---|---|---|
| **Bezeichnung** | **Maxₑₓ** | **Maxₑₘ** | **Bezeichnung** | **Maxₑₓ** | **Maxₑₘ** |
| Alexa Fluor 488 | 495 nm | 519 nm | Alexa Fluor 555 (oder auch Alexa Fluor 546 oder 568) | 555 nm | 565 nm |
| Alexa Fluor 546 | 556 nm | 573 nm | Alexa Fluor 633 | 632 nm | 647 nm |
| Alexa Fluor 555 | 555 nm | 565 nm | Alexa Fluor 647 | 650 nm | 668 nm |
| Alexa Fluor 568 | 578 nm | 603 nm | Cy5 | 649 nm | 670 nm |
| Alexa Fluor 568 (oder auch Cy3) | 578 nm | 603 nm | Alexa Fluor 633 (oder auch Cy5 oder Alexa Fluor 647) | 632 nm | 647 nm |
| Europium (Eu³⁺) | 395 nm 466 nm | 617 nm | Allophycocyanin | 650 nm | 660 nm |
| Eu³⁺ (oder LaF₃) | 395 nm | 591 nm | Alexa Fluor 594 (oder auch Alexa Fluor 610 oder 633) | 590 nm | 630 nm |
| LaPO₄ (oder Ce oder Tb) | 266 nm | 542 nm | Alexa Fluor 532 | 530 nm | 560 nm |
| zyan fluoreszierendes Protein (CFP) | 452 nm | 505 nm | gelb fluoreszierendes Protein (YFP) | 514 nm | 527 nm |
| Luciferase | | | YFP | 514 nm | 527 nm |
| EGFP | 488 nm | 509 nm | YFP | 514 nm | 527 nm |

FRET-Analysen eigen sich auch für das High throughput screening. An den einen Bindungspartner ist ein FRET-Donor an den anderen ein FRET-Akzeptor gebunden, wobei beiden zusammen ein FRET-Paar bilden. Liegen die beiden Bindungspartner unabhängig voneinander *in vitro* oder in der Zelle tritt kein FRET auf. Durch die Interaktion der beiden Bindungspartner werden die FRET-Donor und FRET-Akzeptor in unmittelbare Nachbarschaft gebracht, wodurch der FRET ausgelöst wird.
Durch den FRET nimmt die Strahlungsemission und der Fluoreszenzlebensdauer des Donors ab, sowie die Akzeptoremission zu. Dies resultiert in einer messbaren Verschiebung der Wellenlänge des emittierten Lichts bei Interaktion der Bindungspartner, welche mit Hilfe geeigneter instrumenteller Methoden nachgewiesen werden. Für diesen Zweck eignen sich beispielsweise Fluoreszenzmikroskope, Flow Cytometer, Fluorimeter oder Plattenspektrophotometer.

Die Testsubstanzen beeinflussen die Interaktion der Bindungspartner. Bevorzugt werden Testsubstanzen ausgewählt, welche die Interaktion von SWAP-70 mit STAT-6 verringern und/oder die Interaktion von SWAP-70 mit BCL-6 verringern.

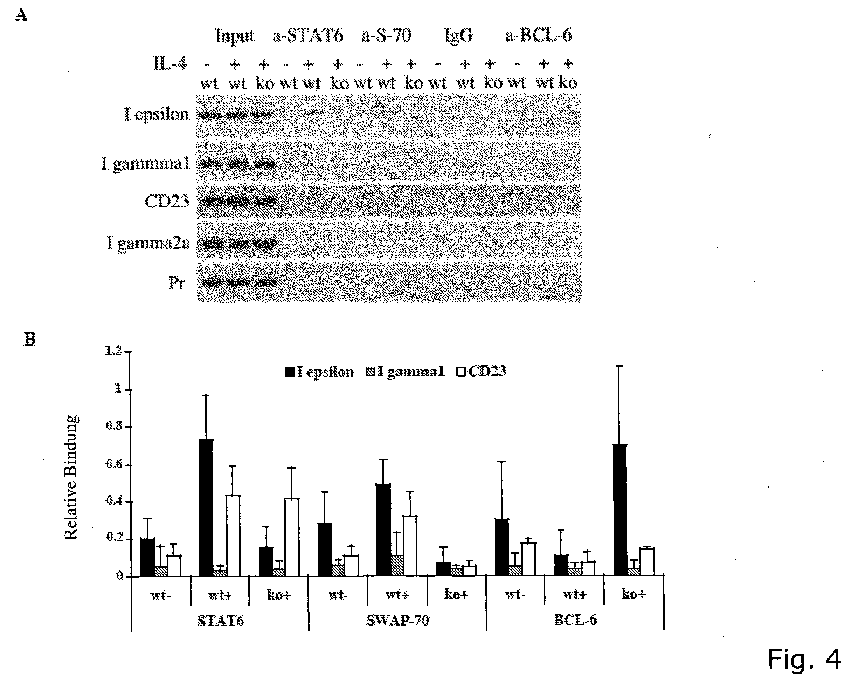

Die Erfinder haben durch Chromatinimmunoprezipitation (ChIP) nachgewiesen, dass SWAP-70 in IL-4 stimulierten B-Zellen zusammen mit STAT-6 und BCL-6 an den Promoterbereich des IgE-Gens (Iε Promoter) bindet (s. Fig. 4A und 4B) jedoch nicht an den Promoterbereich des IgG1-gens (γ1 Promoter). SWAP-70 reguliert dadurch die STAT-6 abhängige Transkription spezifisch am Iε Promoter.

Bevorzugt wird Schritt (i) in Gegenwart eines oder mehrerer regulatorischer Elemente des IgE Gens durchgeführt, an die sowohl STAT-6 und BCL-6 binden. Diese regulatorischen Elemente sind DNA-Sequenzen, die bevorzugt als Doppelstrang vorliegen und den Iε Promoter und/oder folgende Sequenzen enthalten:

### Iε GLT Promoter:

Die STAT-6 Bindungsstelle ist unterstrichen. Sie liegt bei -111 bis -102 relativ zur Startstelle der GLT Transkription des Iε. Die -111/-102 Sequenz ist auch eine von mindestens zwei Bindestellen für BCL-6.

### Die zweite STAT-6 Bindungsstelle liegt bei -157 bis -149:

Sequenz: 5'...TGTCTCCTAGAAAGAGG...3' (SEQ ID Nr. 2).

### Die BCL-6 Bindungsstelle ist unterstrichen.

Bevorzugt enthalten die in der Erfindung verwendeten regulatorischen Elemente die SEQ ID Nr. 1 und 2.

Bevorzugt wird Schritt (iii) eine Testsubstanz ausgewählt wird, welche die Wechselwirkung der beiden Gegenspieler STAT-6 und BCL-6 mit regulatorischen Elementen des IgE Gens zugunsten von BLC6 verschiebt.

SWAP-70 kontrolliert die Bindung von BCL-6 und STAT-6 an regulatorische Elemente des IgE-Gens. Diese Bindung wird bevorzugt *in vitro* nachgestellt, vorzugsweise in einem Electrophoretic mobility shift assay EMSA (auch gel shift oder band shift assay genannt) oder high throughput Varianten davon. Hier wird die Bindung an die regulatorischen Elemente des IgE Gens (d. h. Oligonukleotide die bevorzugt wie oben ausgewählt sind) z. B. im Multiwell-Format getestet. Die Wirkung der Testsubstanz auf die SWAP-70 vermittelte Bindung von STAT-6 und/oder BCL-6 kann anhand der Assoziation entsprechend markierter Bindungspartner (SWAP-70, BCL-6 und/oder STAT-6) und Oligonukleotide nachgewiesen werden. Die Oligonukleotide sind dabei bevorzugt an ein Trägermaterial fixiert. Alternativ werden Oligonukleotide markiert und einer der Bindungspartner an ein Trägermaterial fixiert.

Die mit dem erfindungsgemäßen Verfahren ermittelten Testsubstanzen werden bevorzugt auf die biologische Wirksamkeit, d.h. die Hemmung der IgE Produktion, getestet. Dazu werden bevorzugt B-Lymphozyten *in situ* oder *in vivo* zur IgE-Produktion angeregt. Bevorzugt werden dabei isolierte Milzzellen (z. B. der Maus) verwendet, da diese eine große Zahl an B-Lymphozyten enthalten. Die B-Lymphozyten müssen dazu nicht aufgereinigt werden. Die B-Lymphozyten können mittels IL-4 (bevorzugt murin und z. B. rekombinant hergestellt) und CD40L (z. B. durch fixierte CD40L-exprimiernde Zellen) zur IgE-Produktion angeregt werden. Die IgE-Produktion kann einfach und mit hohem Durchsatz, z. B. mit Hilfe der FACS Analyse oder ELISA, gemessen werden. Bevorzugt werden Testsubstanzen ausgewählt, welche die IgE Produktion reduzieren.

Bevorzugt wird um den Mechanismus der Wirkung der Testsubstanzen zu analysieren eine co-Immunopräzipitationen von SWAP-70 mit BCL-6 oder mit STAT-6 aus B-Zellen durchgeführt, welche wie im obigen Absatz beschrieben zur IgE-Produktion angeregt und mit der Testsubstanz in Kontakt gebracht wurden. Durch die co-Immunopräzipitation lässt sich bestimmen, wie die Testsubstanz die Interaktion von SWAP-70 mit BCL-6 und/oder mit STAT-6 beeinflusst. Inhibiert die die Testsubstanz die Interaktion von SWAP-70 mit STAT-6 werden diese beiden Proteine in geringerer Menge co-präzipitieren. Verstärkt die Testsubstanz die Interaktion von SWAP-70 mit BCL-6 werden diese beiden Proteine in erhöhter Menge co-präzipitieren.

Eine sehr vorteilhafte Variante ist die Chromatin Immuno-Präzipitation. Hier kann die Interaktion von SWAP-70, BCL-6 und/oder STAT-6, bevorzugt aller drei Proteine, mit regulatorischen Elementen des IgE-Gens in der Zelle, d.h. mit hoch relevantem Material *ex vivo,* bestimmt bzw. der Einfluss der Testsubstanz auf diese Interaktion.

Die mit dem erfindungsgemäßen Verfahren identifizierten Substanzen eignen sich insbesondere zur Behandlung von IgE-vermittelten Allergien, insbesondere zur Behandlung von IgE abhängigen allergischen Reaktionen bzw. Typ I Allergien und von IgE-abhängigen Autoimmunerkrankungen. Die in erfindungsgemäßen Verfahren identifizierten Substanzen eignen sich insbesondere zur Behandlung von IgE-abhängigen Erkrankungen wie allergischem Asthma, allergischer Rhinitis, allergischer Konjunktivitis, allergischen Reaktionen des Gastrointestinaltrakts (insbesondere Erbrechen und Durchfälle), Neurodermitis, Psoriasis, Kontaktekzem, Urtikaria, allergischen Ödemen, insbesondere dem Larynxödem und dem Angiödem (Quincke-Ödem), anaphylaktischem Schock, allergischer Vaskulitis, Angiitis oder Granulomatose (Churg-Strauss-Syndrom), Hyper-IgE-Syndrom, Omenn-Syndrom, einige Formen des 22q11-Syndroms, der rheumatoiden Arthritis, des Lupus erythematodes, der Typ I Diabetes, des Sjogren's Syndrom, und des bullösen Pemphigoids.

Gegenstand der Erfindung ist auch ein Testkit gemäß Anspruch 7 zur Identifizierung einer Substanz (Testsubstanz), welche die IgE-Produktion hemmen, enthaltend:
(a) SWAP-70 und
(b) BCL-6 und/oder STAT-6
(c) ggf. regulatorische Elemente des IgE Gens.

Für *in vitro* Assays liegen die Komponenten (a) und (b) jeweils als isolierte Proteine und die optionale Komponente (c) als isolierte DNA vor.

Die Proteine und die DNA sind bevorzugt wie oben beschrieben an einen Trägermaterial gebunden oder mit einem Marker versehen.

Für *in situ* oder *in vivo* Assays liegen bevorzugt alle Komponenten (d. h. (a), (b) und ggf. (c)) in einer Zelle oder einem multizellulären Organismus, den Menschen ausgenommen, vor.

Die Proteine, also Komponenten (a) und (b), sind hier wie oben beschrieben mit einem Marker versehen, der bevorzugt ausgewählt ist aus Affinititätstags, Haptameren, fluoreszierenden oder chemiluminizierenden Biomoleküle. Die Markierung erfolgt hier bevorzugt durch Expression als Fusionsprotein (Protein + Marker).

Bevorzugt werden wie oben beschrieben zwei Marker ausgewählt die zur Erzeugung eines Förster-Resonanzenergietransfers (FRET) geeignet sind.

Die Erfindung wird nachfolgend durch Abbildungen und Beispiele näher erläutert ohne auf diese beschränkt zu sein:

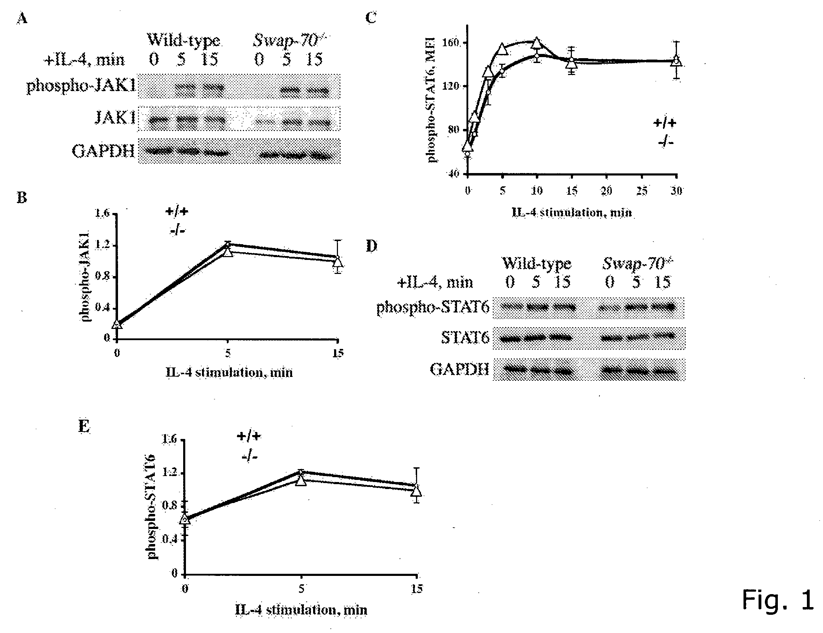

**Fig. 1** zeigt, dass IL-4 Stimulierung die Kinase JAK1 sowohl in Wildtyp als auch *Swap-70*^{*-*/*-*} B-Zellen aktiviert (Fig. 1A, 1B), und dass STAT-6 ebenfalls in Wildtyp als auch *Swap-70*^{*-*/*-*} B-Zellen aktiviert wird (Fig. 1C, 1D, 1E). MFI = Mittlere Fluoreszenzintensität.

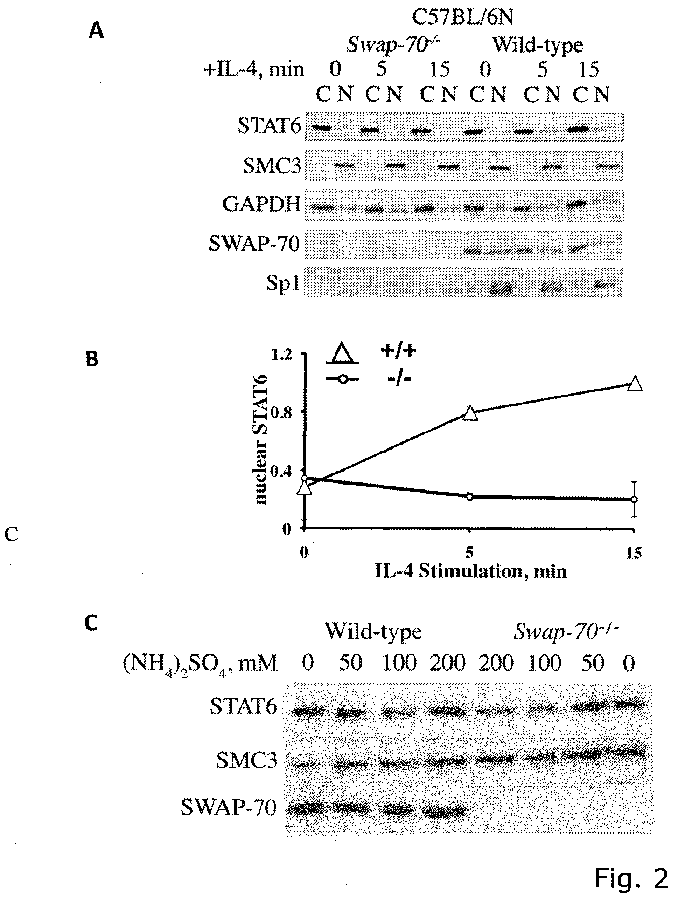

**Fig. 2A** und **2B** zeigen die in *Swap-70*^{*-*/*-*} B-Zellen reduzierte Akkumulation von STAT-6 in Kernextrakten der mit IL-4 aktivierten B-Zellen. C = cytoplasmatischer Extrakt; N = nukleärer Extrakt; min = Minuten Stimulierung. **Fig. 2C** zeigt Extraktion von STAT-6 aus Kernen IL-4 aktivierter B-Zellen mit schrittweise ansteigender Salzkonzentration (Ammoniumsulfat). Als Vergleichsproteine dienen SMC3 und SWAP-70. Aus *Swap-70*^{*-*/*-*} B-Zellkernen kann deutlich weniger mit hoher Affinität bindendes, d.h. bei hoher Salzkonzentration eluierendes, STAT-6 extrahiert werden (200 mM Proben). Sp1 = Sp1 transcription factor.
**Fig. 3** (Floureszenzmikroskopie) zeigt, dass die generelle Lokalisation von STAT-6 im Zellkern nicht beeinträchtigt ist. Oben: STAT-6; Unten: DAPI-Färbung der Zellkerne; Mitte: GFP identifiziert *Swap-70*^{*-*/*-*} Zellen. Wildtyp und *Swap-70*^{*-*/*-*} Zellen liegen hier nebeneinander vor.
**Fig**. **4A** und **4B** zeigen Ergebnisse einer Chromatinimmunopräzipitation mit anti-STAT-6 (a-STAT-6), anti-SWAP-70 (a-S-70), IgG (Kontrolle) und anti-BCL-6 (a-BCL-6) nach IL-4 Stimulation sowohl in Wildtyp (wt) als auch *Swap-70*^{*-*/*-*} (ko) B-Zellen. Input = Chromatinstartmaterial. Die Ergebnisse zeigen, dass in Abwesenheit von SWAP-70 deutlich mehr BCL-6 und deutlich weniger STAT-6 an den Is Promoter rekrutiert werden, sowie dass SWAP-70 weitgehend unabhängig von der IL-4 Stimulation an den Iε-Promoter rekrutiert wird. 4B stellt die Quantifizierung dieser Ergebnisse dar.

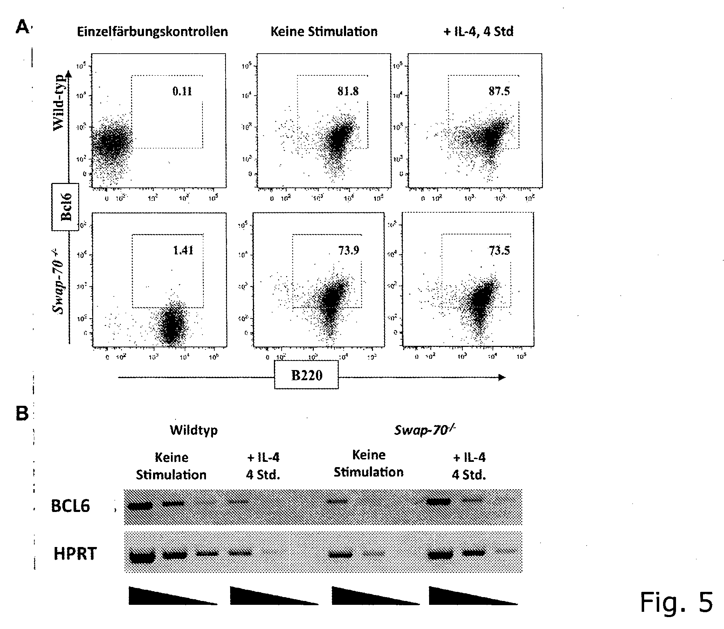

**Fig. 5** zeigt, dass BCL-6 in wt und *Swap-70*^{*-*/*-*}-B-Zellen etwa gleichermassen exprimiert wird. Fig. 5A zeigt eine FACS-Analyse IL-4 stimulierter B-Zellen (B220+) hinsichtlich der BCL-6 Expression. Fig. 5B zeigt die Analyse der BCL-6 mRNA mit Hilfe semiquantitativer RT-PCR. Jeweils werden wt und *Swap-70*^{*-*/*-*} Zellen mit und ohne IL-4 Stimulus, bzw. deren mRNA Präparationen, miteinander verglichen und in Relation zur Ladekontrolle HPRT gesetzt.

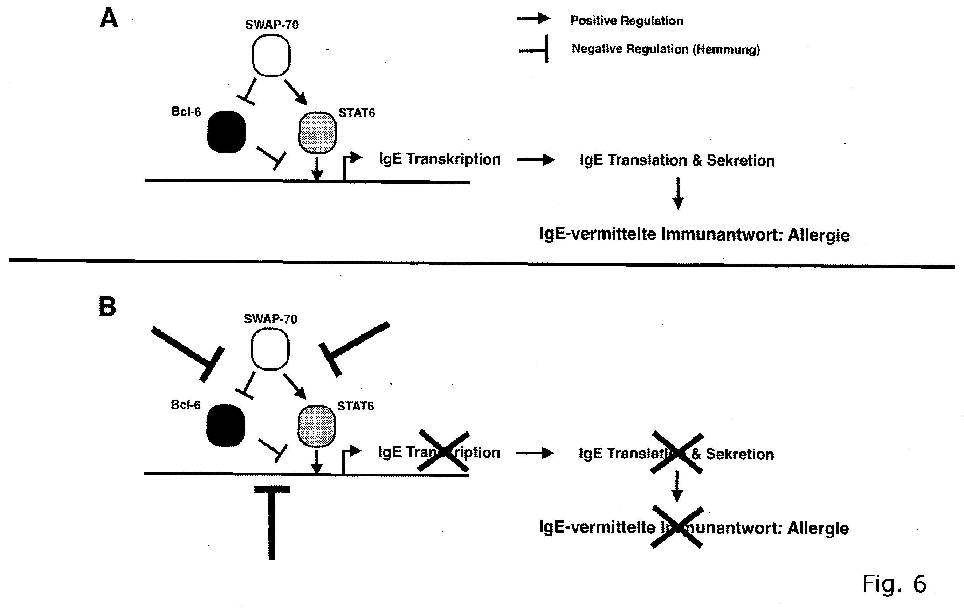

**Fig. 6** zeigt ein Schema der SWAP-70 medierten Regulierung der IgE-Produktion durch Beeinflussung des STAT-6/BCL-6-Gleichgewichts. A: Situation in B Lymphocyten nach IL-4 Stimulierung. B: mögliche Ansatzpunkte der pharmakologischen Hemmung, dargestellt im dick gedruckten Hemmungssymbol. Die Aufhebung der SWAP-70 abhängigen Blockierung von BCL-6 verstärkt die inhibitorische Wirkung von BCL-6 auf die IgE-Produktion. Die Aufhebung der SWAP-70 abhängigen Stimulierung von STAT-6 reduziert die stimulierende Wirkung von STAT-6 auf die IgE-Produktion.

### Materialien und Methoden

Zellisolierung: B Lymphozyten wurden aus der Milz von Wildtyp (wt) oder *Swap-70*^{*-*/}*⁻ Mäusen* (4 bis 8 Wochen alt C57BL/6) mit Standardmethoden und negativer Selektion mit MACS beads (Miltenyi Biotec) isoliert. B Lymphozyten oder Gesamtmilzzellen (0.3-0.5x10⁶ Zellen/ml) wurden in Gegenwart von 1,6 bis 3 ng/ml IL-4 (aus X63-IL-4 Zellkultur-Überstand) kultiviert. Wenn angeben wurden die Zellen auf einem Rasen von L47 Fibroblasten-Zellen kultiviert, die CD40L auf der Oberfläche exprimieren.

Durchflusszytometrie: Der durchflusszytometrische Nachweis erfolgte mit Standardmethoden mit anti-Maus CD40R-PE, anti-Maus IgE-FITC (R35-72), anti-Maus IgG1-PE (A85-1), anti-Maus CD45RB220-PE (RA3-6B2), CD45R/B220-PerCP (RA3-6B2) (BD Biosciences) oder anti-Maus IL-4R-biotinyliert (R&D Systems). Die Oberflächen-Ig-Produktion wurde nach Stripping (1 min, 85 mM NaCl, 5 mM KCl, 10mM EDTA, 50 mM NaCH3COO, pH 4,0) gemessen; Phospho-STAT-6 mit anti-Maus phospho-STAT-6 (Y641)(J71-773.58.11) (BD Biosciences) nach Zellpermeabilisierung (90 % Methanol) zur Färbung genutzt.

**Real-time RT-PCR: Der Nachweis von IgG1 bzw. IgE mRNA Transkription erfolgte in CD40L/IL-4-stimulierten Milzellen bzw. isolierten B-Zellen durch Real-time RT-PCR. Die Expression von GAPDH mRNA wurde als Standard gemessen. Verwendete Primer für die PCR:**

| Name | Sequenz | Produktlänge | SEQ ID Nr. |
|---|---|---|---|
| | | | |
| GLTγ1 Fwd | 5'-TCGAGAAGCCTGAGGAATGT-3' | | 3 |
| GLTγ1 Rev | 5'-ATAGACAGATGGGGGTGTCG-3' | 100bp | 4 |
| | | | |
| GLTε□Fwd | 5'-CTGGCCAGCCACTCACTTAT-3', | | 5 |
| GLTε□Rev | 5'-CAGTGCCTTTACAGGGCTTC-3' | 100bp | 6 |
| | | | |
| GAPDH Fwd | 5'-CACAGGACTAGAACACCTGC-3' | | 7 |
| GAPDH Rev | 5'-GCTGGTGAAGACCTCT-3' | 248bp | 8 |

Immunoblots (IB): Gesamtproteinextrakte (total cell extracts), sowie Kernprotein- und Zytoplasmaproteinextrakte wurden mittels Standardmethoden aus Milzellen oder isolierten B-Zellen erhalten. Die Proteine in den erhaltenen Proteinextrakten wurden jeweils mittels SDS-Page aufgetrennt und auf eine Nitrozellulosemembran geblottet. Verwendete Antikörper: anti-GAPDH (monoklonal 6C5, Santa Cruz Biotechnology), anti-JAK1 (rabbit anti-Maus), anti-phosho-JAK1 (rabbit anti-Maus, Cell Signaling Technology), anti-STAT-6 (monoklonal YE361), antiphosho-STAT-6 (monoklonal Y641, Abcam), anti-SMC3 (rabbit anti-Maus), anti-Sp1 (rabbit anti-Maus, affinitätsgereinigt, Bethyl Laboratories), anti-SWAP-70 (rabbit anti-Maus, affinitätsgereinigt).

Immunofluoreszenz (IF): Der Nachweis von SWAP-70 und STAT-6 Protein erfolgte per Immunofluoreszenz in MACS aufgereinigten B Lymphozyten (1-2x106 Zellen/m), die mit IL-4 oder IL-4 und CD40L stimuliert wurden, mit anti-STAT-6 (1:100 Abcam) bzw. anti-SWAP-70 (rabbit anti-mouse, affinitätsgereinigt 1 µg/ml) und goat-anti-rabbit-Cy3 (1:500) (Dianova) als sekundärem Antikörper.

Chromatin-Immunoprezipitation (ChIP): MACS-aufgereinigte B Lymphozyten (2x10⁶ Zellen/ml, aus wt or Swap-70^{-/-} Mäusen) wurden für 4 h mit IL-4 (2 ng/ml) stimuliert und dann nach Fixierung (1 % Formaldehyd, 10 min, RT) durch ChIP-Puffer (1 % SDS, 10 mM EDTA, 50 mM Tris HCl, pH8.1, Proteaseinhibitorcocktail) und Ultraschall aufgeschlossen. Nach Zentrifugation wurde ein Überstand mit DNA-Fragmenten in der Größe von 100 bis 500 bp erhalten. Der Überstand wurde mit blockierten Protein A Beads (Protein A Beads Agarosebeads blockiert mit 0,2 mg/ml Lachssperma-DNA and 0,5 mg/ml BSA) gereinigt und anschließend mit 1 µg anti-SWAP-70, 2 µg anti-STAT-6 (M20), 2 µg anti-BCL-6 (N-3) oder 1 µg Rabbit IgG (Santa Cruz Biotech.) inkubiert (4 °C über Nacht). Die so erhaltenen Proben wurden mit blockierten Protein A Beads inkubiert (4 °C, 1 h). Die Beads wurden gewaschen und eluiert (100 mM NaHCO3, 1 % SDS), 6 h bei 65 °C inkubiert und mit Proteinase K (1 h, 45 °C) behandelt. DNA wurde isoliert und eine PCR mit folgenden Primerpaaren wurde durchgeführt:

**Iε -Promoter (I epsilon):**

| Name | Sequenz | Produktlänge | SEQ ID Nr. |
|---|---|---|---|
| I epsilon Fwd | 5'-CTAGAAAGAGGCCTACACCTG-3' | | 9 |
| I epsilon Rev | 5'-CCAGACTGTTCTTATTCG-3' | 243b | 10 |

**Iγ1 -Promoter (I gamma1):**

| Name | Sequenz | Produktlänge | SEQ ID Nr. |
|---|---|---|---|
| I gamma1 Fwd | 5'-AGGGGGTGAGGGGGAGTCCA-3' | | 11 |
| I gamma1 Rev | 5'-CCCCCAAAGGCCCAGGTGC-3' | 145 bp | 12 |

**CD23-Promoter:**

| Name | Sequenz | Produktlänge | SEQ ID Nr. |
|---|---|---|---|
| CD23 Fwd | 5'-TGGCATCGCTGACTCTCCAACA-3' | 118bp | 13 |
| CD23 Rev | 5'-CTGGGTGGCCACAGCACACA-3' | | 14 |

**Kontrollpromoter (ohne STAT-6-Bindingsstelle):**

| Name | Sequenz | Produktlänge | SEQ ID Nr. |
|---|---|---|---|
| Pr Fwd | 5'-TTTGAACTGGAGCTCAGCTGG-3' | | 15 |
| Pr Rev | 5'-TCCATGGTGCTAGCCATATGC-3' | 160 bp | 16 |
| | | | |
| I gamma2a Fwd | 5'-CTGTCACCCACTTTCAATCCTG-3' | | 17 |
| I gamma2a Rev | 5'-GCGTGAAGAAGATTGCTGCTATT-3' | 148 bp | 18 |

### Ergebnisse:

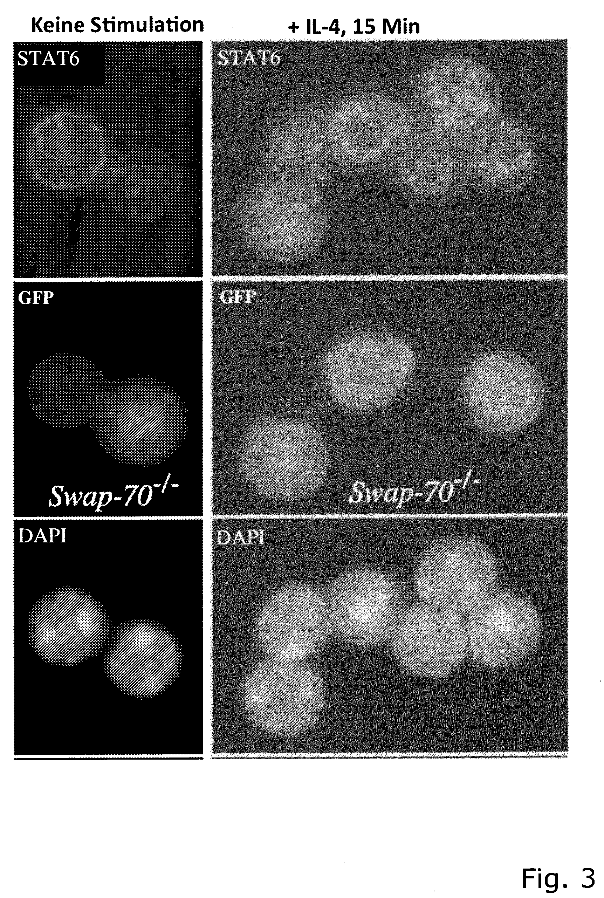

Die Ergebnisse der oben aufgeführten Versuche werden wie folgt zusammengefasst:
Die Zellfraktionierung von wt oder Swap-70^{-/-}-Milzzellen zeigt, dass STAT-6 als Antwort auf die IL-4 Stimulierung in wt Kernextrakten, die durch Salzextrahierung von isolierten Kernen hergestellt wurden, akkumuliert. Jedoch enthalten Kernextrakte von Swap-70^{-/-}-Milzzellen weniger STAT-6 als Wildtyp-Milzzellen (Fig. 2A, 2B). Ähnlich wie STAT-6 (Fig. 3) akkumuliert SWAP-70 im Kern durch Stimulierung durch IL-4 oder IL-4 kombiniert mit CD40L. Der Nachweis von STAT-6 in wt und swap-70^{-/-}-B-Zellen durch IF nach Fixierung und Permeabilisation der Zellen ("Input") zeigte keinen offensichtlichen Unterschied zwischen IL-4 aktivierten wild type- und Swap-70^{-/-}-Zellen. Dies kann darauf hinweisen, dass STAT-6 effizient in die Swap-70^{-/-}-Zellkerne translokalisiert und somit experimentell fixiert und dort sichtbar gemacht werden kann, aber dass es sich nicht so eng mit dem Chromatin verbindet und deshalb in Aufbereitungen von Kernextrakten verloren geht. Aus diesen Daten lässt sich schlussfolgern, dass STAT-6 in Abwesenheit von SWAP-70 eine niedrige Affinität zum Is-Promotor aufweist und dass die Anwesenheit von SWAP-70 diese Affinität erhöht. STAT-6 bindet mit unterschiedlicher Affinität an unterschiedliche Promotoren und Bindung an Iε erfordert eine hohe Affinität.

Um die Stärke der Assoziation von STAT-6 mit Chromatin bei Anwesenheit oder Abwesenheit von SWAP-70 zu messen, haben die Erfinder Zellen durch Dounce-Homogenisierung zertrennt, um ein durch Detergenz induziertes Auslaufen von locker gebundenem STAT-6 aus den Kernen zu vermeiden. Steigende Salzkonzentrationen wurden verwendet um Proteine sequentiell aus den Kernen von IL-4 aktivierten Splenozyten zu extrahieren (Fig. 2C). Vergleichbare Fraktionen von STAT-6 dissoziierten vom Chromatin in den Kernextrakt bei Konzentrationen von 0 mM und 50 mM Ammoniumsulfat von wt und Swap-70^{-/-} Kernen. Diese Fraktionen stammen aus nukleoplasmatischem STAT-6, welches nicht oder mit geringer Affinität an Chromatin gebunden ist. Die Menge von STAT-6, die aus Swap-70^{-/-}-Zellkernen durch die Zugabe von 200 mM Ammoniumsulfat extrahiert wurde, war jenoch bedeutend geringer als jene, die aus wt Kernen extrahiert wurde. Dies weist generell auf eine schwächere Bindung von STAT-6 mit Chromatin in Abwesenheit von SWAP-70 hin. Da die Bindung an Iε eine hohe Affinität erfordert, ist STAT-6 anscheinend an diesem Promotor deutlich reduziert oder sogar nicht vorhanden.

Um dies nachzuweisen, führten die Erfinder Chromatin Immuno Precipitationen (ChIP) auf wt oder Swap-70^{-/-}-B-Zellen durch, die mit IL-4 stimuliert waren, unter Nutzung von Antkörpern gegen SWAP-70 oder STAT-6. Nicht-stimulierte wt B-Zellen dienten als negative Kontrolle. Primer, die spezifisch für die Iε Region mit der STAT-6 Bindungsstelle sind, wurden für die semi-quantitative PCR-Analyse der DNA genutzt, die mit SWAP-70 oder STAT-6 präzipitierte. Die Ergebnisse zeigen, dass STAT-6 in Wildtyp-Zellen nur nach IL-4-Behandlung an die spezifische Is Promotorregion (I epsilon) bindet (Fig. 4A, 4B). In Swap-70^{-/-}-B-Zellen (ko) stimuliert mit IL-4 ist die Verbindung von STAT-6 mit Iε stark reduziert (Fig. 4A, 4B). Eine Verbindung von STAT-6 mit Iγ1 (I gamma1) war nicht erkennbar, was auf eine schwache und/oder nur vorübergehende Interaktion von STAT-6 mit dem γ1 Promotor hinweist. Kontrollen, die Promotor-Regionen von IgG2a (I gamma2 - Iγ2a) und eines Proteasom-Gens (Pr), mit fehlenden STAT-6 Bindungsorten, haben keine PCR-Signale erzeugt (Fig. 4A, 4B). CD23 und der IL4R sind für ihre Transkribierung unter der Kontrolle von STAT-6 bekannt. Swap-70^{-/-}-B-Zellen zeigen eine normale Induktion der CD23-Expression als Antwort auf entweder IL-4- oder CD40-Stimulierung durch CD40L oder nach Behandlung mit beiden Stimulanzen, sowie eine mit dem Wildtyp vergleichbare IL-4Rα Hochregulierung. Dies belegt die SWAP-70-unabhängige STAT-6-Kontrolle der CD23- und IL4R-Promotoren. Somit reguliert SWAP-70 vorrangig die STAT-6-abhängige Transkription am Iε-Promotor. Die Bindung von STAT-6 an den CD23-Promotor wurde zur Kontrolle getestet. In ChIP mit wt und Swap-70^{-/-}-B-Zellen wurde eine vergleichbare Rekrutierung von STAT-6 an den CD23 Promotor beobachtet (Fig. 4A, 4B).

ChIP mit anti-SWAP-70 Antikörpern (a-S-70) belegt dass SWAP-70 an den Iε-Promotor in wt-Zellen in einer IL-4-abhängigen Art rekrutiert wird (Fig. 4A, 4B). Somit bindet SWAP-70 direkt oder indirekt den Iε-Promotor.

BCL-6 ist bekannt dafür, dass es die Bindung von STAT-6 an den Iε-Promotor antagonisiert. BCL-6 und STAT-6 binden an überlappende Sequenzen innerhalb des Is-Promotor. BLC6 konkurriert daher mit STAT-6 und hemmt die Transkription durch die Rekrutierung von Co-Repressoren. Die Erfinder bestimmten, ob die Abwesenheit von STAT-6 mit einer größeren Anwesenheit von BCL-6 am Iε Promotor korreliert. Während in wt Zellen ChIP-Daten eine reduzierte BCL-6 Bindung nach IL-4-Stimulierung zeigen, ergibt sich eine deutlich erhöhte Bindung von BCL-6 an den Is Promotor in IL-4 aktivierten SWAP-70^{-/-}-B-Zellen verglichen mit wt. Da BCL-6 RNA- und Proteinexpression in Swap-70^{-/-}-B-Zellen der in wt B-Zellen (Fig. 5) entsprechen, belegt dies die Kontrolle der BCL-6-Bindung an den Iε-Promotor durch SWAP-70.

BCL-6 reguliert nicht die CD23-Transkription. Dementsprechend wurden keine CD23-spezifischen ChIP-Signale mit verschiedenen anti-BCL-6-Antikörpern erhalten.

Insgesamt zeigen die Erfinder damit, dass SWAP-70 für die IL-4 induzierte ε germline Transkription (GLT) benötigt wird, wobei SWAP-70 die kritische Balance zwischen BCL-6 und STAT-6 am Iε-Promotor kontrolliert. Es kann geschlussfolgert werden, dass die Wechselwirkung von SWAP-70 mit BCL-6 und/oder dem Iε-Promotor verhindert, dass BCL-6 die STAT-6-medierte Induktion von ε GLT blockiert. Die Erkenntnis, dass SWAP-70 somit ein übergeordneter Regulator für BCL-6 und STAT-6 in der spezifischen Regulation der IgE Produktion ist, erlaubt es, gezielt in diesen neuen Stoffwechselweg einzugreifen und somit gezielt die Produktion von IgE, welches ein Schlüsselmolekül in Allergien und vielen Autoimmunkrankheiten ist, zu hemmen.

## Patentansprüche

1. Verfahren zur Identifizierung einer Substanz, welche die IgE-Produktion hemmt, wobei das Verfahren folgendes umfasst:
(i) Inkontaktbringen:
(a) mindestens einer Testsubstanz mit
(b) SWAP-70 und
(c) BCL-6 und/oder STAT-6
(ii) Bestimmung der Interaktion der Bindungspartner *in vitro, in situ* oder *in vivo,* wobei bei Messung der Interaktion in Vertebraten der Mensch ausgenommen ist,
(iii) Auswahl einer Testsubstanz, welche die SWAP-70 medierte Aktivierung von STAT-6 spezifisch inhibiert und/oder die BCL6 medierte Inhibierung der SWAP-70 medierten Aktivierung von STAT-6 verstärkt.

2. Verfahren nach Anspruch 1, **dadurch gekennzeichnet, dass** eine Testsubstanz ausgewählt wird, welche die Interaktion von SWAP-70 mit STAT-6 hemmt.

3. Verfahren nach Anspruch 1 oder 2, **dadurch gekennzeichnet, dass** eine Testsubstanz ausgewählt wird, welche die Interaktion von SWAP-70 mit BCL-6 so beeinflusst, dass die Wirkung von BCL6 verstärkt wird.

4. Verfahren nach einem der Ansprüche 1 bis 3, **dadurch gekennzeichnet, dass** Schritt (i) in Gegenwart von regulatorischen Elementen des IgE Gens durchgeführt wird an die sowohl STAT-6 und BCL-6 binden.

5. Verfahren nach Anspruch 4, **dadurch gekennzeichnet, dass** eine Testsubstanz ausgewählt wird, welche die Wechselwirkung der beiden Gegenspieler STAT-6 und BCL-6 mit regulatorischen Elementen des IgE Gens zugunsten von BLC-6 verschiebt.

6. Verfahren nach einem der Ansprüche 1 bis 5, **dadurch gekennzeichnet, dass** die Interaktion der Proteine und ggf. der regulatorischen Elemente des IgE Gens *in vitro* oder *in situ* gemessen wird.

7. Testkit zur Identifizierung einer Substanz, welche die IgE-Produktion hemmen, enthaltend:
(a) SWAP-70 und
(b) BCL-6 und/oder STAT-6
(c) ggf. regulatorische Elemente des IgE Gens
wobei
i. entweder die Komponenten (a) und (b) jeweils als isolierte Proteine und die Komponente (c) als isolierte DNA vorliegen oder
ii. alle Komponenten in einer Zelle oder einem nicht menschlichen multizellulären Organismus vorliegen und die Komponenten (a) und (b) mit einem Marker versehen sind.

## Claims

1. Method for identifying a substance which inhibit the IgE production, the method comprising the following:
(i) contacting:
(a) at least one candidate substance with
(b) SWAP-70 and
(c) BCL-6 and/or STAT-6
(ii) detection of the interaction of the binding partners *in vitro, in situ* or *in vivo, ,* whereat measuring the interaction in vertebrates excludes humans,
(iii) selecting a candidate substance which specifically inhibits the SWAP-70 mediated activation of STAT-6 and/or reinforces the BCL6 mediated inhibition of the SWAP-70 mediated activation of STAT-6.

2. Method according to claim 1, **characterized in that** a candidate substance is selected which inhibits the interaction of SWAP-70 with STAT-6.

3. Method according to claim 1 or 2, **characterized in that** a candidate substance is selected which affects the interaction of SWAP-70 with BCL-6 such that the effect of BCL6 is reinforced.

4. Method according to one of the claims 1 to 3, **characterized in that** step (i) is carried out in the presence of regulatory elements of the IgE gene to which STAT-6 as well as BCL-6 bind.

5. Method according to claim 4, **characterized in that** a candidate substance is selected which shifts the interaction of the two antagonists STAT-6 and BCL-6 with regulatory elements of the IgE gene in favor of BLC-6.

6. Method according to one of the claims 1 to 5, **characterized in that** the interaction of the proteins and optionally of the regulatory elements of the IgE gene is measured *in vitro* or *in situ.*

7. Test kit for identifying a substance which inhibit the IgE production, containing:
(a) SWAP-70 and
(b) BCL-6 and/or STAT-6
(c) optionally, regulatory elements of the IgE gene
wherein
i. either the components (a) and (b) each are present as isolated proteins and the component (c) as isolated DNA, or
ii. all components are present in a cell or a non-human multicellular organism and components (a) and (b) bear a marker.

## Revendications

1. Procédé destiné à identifier une substance, laquelle inhibe la production d'IgE, le procédé comprenant :
(i) la mise en contact
(a) d'au moins un substance de test avec
(b) du SWAP-70 et
(c) du BCL-6 et/ou du STAT-6
(ii) la détermination de l'interaction des partenaires de liaison *in-vitro, in-situ* ou *in-vivo,* lors de la mesure de l'interaction sur des vertébrés, l'homme étant exclu,
(iii) le choix d'une substance de test, laquelle inhibe spécifiquement l'activation du STAT-6 apportée par le SWAP-70 et/ou renforce l'inhibition apportée par le BCL-6 de l'activation du STAT-6 apportée par le SWAP-70.

2. Procédé selon la revendication 1, **caractérisé en ce qu'**on choisit une substance de test, laquelle inhibe l'interaction du SWAP-70 avec le STAT-6.

3. Procédé selon la revendication 1 ou la revendication 2, **caractérisé en ce qu'**on choisit une substance de test, laquelle influence l'interaction de SWAP-70 avec BCL-6, de sorte à renforcer l'effet du BCL-6.

4. Procédé selon l'une quelconque des revendications 1 à 3, **caractérisé en ce qu'**on réalise l'étape (i) en présence d'éléments régulateurs du gène IgE auxquels se lient aussi bien le STAT-6 que le BCL-6.

5. Procédé selon la revendication 4, **caractérisé en ce qu'**on choisit une substance de test laquelle déplace l'interaction des deux antagonistes STAT-6 et BCL-6 avec des éléments régulateur du gène IgE en faveur du BLC-6.

6. Procédé selon l'une quelconque des revendications 1 à 5, **caractérisé en ce qu'**on mesure *in vitro* ou *in situ* l'interaction des protéines et le cas échéant des éléments régulateurs de l'IgE.

7. Kit de test destiné à identifier une substance, laquelle inhibe la production d'IgE, contenant :
(a) du SWAP-70 et
(b) du BCL-6 et/ou du STAT-6
(c) le cas échéant des éléments régulateurs du gène IgE
i. soit les composants (a) et (b) étant chacun présent sous la forme d'une protéine isolée et le composant (c) sous la forme d'un ADN isolé ou
ii. tous les composants étant présents dans une cellule ou dans un organisme multicellulaire non humain et les composants (a) et (b) étant munis d'un marqueur.
